# EUROPEAN PATENT APPLICATION

(11) **EP 2 606 965 A1**
(43) Date of publication of application: **26.06.2013**
(21) Application number: 11194577.0
(22) Date of filing: 20.12.2011
(51) Int. Cl.: B01J 21/10, B01J 23/02, B01J 27/10, C07C 5/48, C07C 17/02

(54) **Catalyst, process for catalyst manufacture and process for catalytic oxidative dehydrogenation**

(71) Applicant: SOLVAY SA, 1120 Bruxelles (BE); Technische Universität München, 80333 München (DE)
(72) Inventor: Gärtner, Christian Achim, 80939 München (DE); Cornelis van Veen, André, 85748 Garching (DE); Lercher, Johannes August, 85521 Ottobrunn (DE)
(74) Representative: Vande Gucht, Anne

(57) **Abstract**

The present invention relates to a catalyst, a process for the manufacture of said catalyst and a process for catalytic oxidative dehydrogenation using said catalyst. The catalyst comprises LiCl (A) and one or more chlorides being different from LiCl (B) on a support (C) which comprises a lanthanide oxide different from dysprosium oxide.

## Description

The present invention relates to a catalyst, a process for the manufacture of said catalyst and a process for catalytic oxidative dehydrogenation using said catalyst.

Lower alkenes, such as ethene and propene, are important starting materials for the production of petrochemicals and fine chemicals. Presently, ethene can be industrially obtained from thermal steam cracking of ethane which does not involve catalysis translating to an expensive high temperature production process with limited energy efficiency and frequent production interruption. The process is strongly endothermic and has to be performed at temperatures around and above 800°C. Furthermore, several products are formed (various saturated and unsaturated hydrocarbons) leading to the necessity of a complex separation of product streams. Severe coke formation leads to further problems, as the heat transfer from the external heat source to the reaction medium is deteriorated. Thus, a selective burning of the coke is necessary in regular intervals. An additional problem is that nitrous oxides (NOx) are formed due to the very high reaction temperatures in the external burners.

Alternatives for the production of lower alkenes are catalytic processes, one of which being catalytic dehydrogenation of lower alkanes. However, the dehydrogenation´s thermodynamic constraints lead to unfavorable low alkane conversion. Furthermore, coking is a major problem of the process which causes rapid catalyst deactivation.

Another catalytic process for the production of lower alkenes is oxidative dehydrogenation (ODH) of lower alkanes. ODH is exothermic and can be operated at temperatures ≤650°C, which can reduce the required energy input. Involving catalysis, specifically in ODH of ethane, could thus yield a highly energy efficient process with long continuous plant operation and a very narrow product spectrum. Provided suitable stable catalysts with ideal selectivity are available for ODH of ethane, the reaction forms ethene and water as products. Hence, the process is in principle suitable to selectively produce ethene without complex separation of products given that the by-produced water can be condensed out easily.

Catalysts activating ethane effectively, while maintaining high ethene selectivity are highly desirable.

Several classes of catalysts have been reported in the literature for ODH of alkanes. Most common catalysts for ODH of ethane are composed of reducible metal oxides, i.e. MoVNb mixed oxides. Those catalysts allow producing mainly ethene, but their activity decreases when catalyst poisons are present in the feed streams.

Supported alkali metal oxide catalysts belong to another class of catalysts active for ODH, particularly also for ODH of ethane. Activity and selectivity could be improved doping these catalysts with secondary chloride salts. Furthermore, the activity could be improved by doping the support with Dy₂O₃ (Gaab, S.; Find, J.; Mueller, T. E.; Lercher, J. A., Kinetics and mechanism of the oxidative dehydrogenation of ethane over Li/Dy/Mg/O/(Cl) mixed oxide catalysts. Top. Catal. 2007, 46, 101; Conway, S. J.; Wang, D. J.; Lunsford, J. H., Selective oxidation of methane and ethane over Li+-MgO-Cl- catalysts promoted with metal oxides. Applied Catalysis A: General 1991, 79, L1).

Catalysts with binary combinations of LiCl and an alkali (sodium or potassium) chloride or an earth alkali (strontium or barium) chloride on Dy₂O₃/MgO supports were also tested in ODH of ethane. Catalysts containing additional alkali (sodium or potassium) or alkaline earth metal (strontium or barium) chlorides besides LiCl exhibited a high selectivity to ethene once reaction temperatures exceed the melting point of the salt combinations, however, at lower activity for ODH of ethane as compared to the case when only LiCl was deposited on the support (Tope, B.; Zhu, Y.; Lercher, J. A. Oxidative dehydrogenation of ethane over Dy2O3/MgO supported LiCl containing eutectic chloride catalysts. Catalysis Today 2007, 123, 11).

To meet the industrial needs of lower alkenes, i.e. C2-C4-alkenes, particularly ethene and propene, there is a demand for new catalysts applicable in ODH. Improvements are desired in various aspects of the catalysts, such as e.g. activity, selectivity, stability of the catalyst, applicability under certain reaction conditions, e.g. resilience against catalyst poisons being present, ease of manufacturing etc.

The present invention thus provides as a first aspect a new catalyst as defined in the claims. It has surprisingly been found that the catalyst according to the present invention resolves aforementioned and other problems. In particular, the present invention provides a new catalyst that is active and selective in ODH, particularly in ODH of ethane to ethene. A catalyst for the selective production of ethene from ethane is thus part of the present invention. The new catalyst enables the production of ethene with high ethene selectivities and high ethane conversion levels.

Surprisingly, it was furthermore found that the catalyst of the present invention is not poisoned by catalyst poisons such as chlorinated hydrocarbons, particularly 1,2-dichloroethane. While, e.g., 1,2-dichloroethane is known to notably deteriorate the catalytic performance of several catalysts of the literature, no deactivation occurs for the catalyst of the present invention, but catalytic activity is even boosted. The novel catalysts are thus suitable for ODH processes, wherein a chlorine containing hydrocarbon is present in the gas phase contacting the catalyst during the process. The chlorine containing hydrocarbon is particularly 1,2-dichloroethane (DCE) and more specifically, DCE coming from a gas stream recycled in a DCE manufacturing process including an ODH step of ethane to ethene.

The catalyst of the present invention comprises LiCl (A) and one or more chlorides being different from LiCl (B) on a support (C) which comprises a lanthanide oxide different from dysprosium oxide. Although dysprosium oxide is working fairly, it is expensive.

In one embodiment, the lanthanide is selected from lanthanum oxide, neodymium oxide, samarium oxide, cerium oxide and/or holmium oxide.

In one sub-embodiment, the lanthanide oxide is selected from lanthanum oxide, neodymium oxide, samarium oxide and/or cerium oxide which are all cheaper than dysprosium oxide but are performing fairly as well, especially in ODH reactions of ethane to ethene. Good results have been obtained when the lanthanide oxide is selected from lanthanum oxide and/or a neodymium oxide, preferably lanthanum oxide.

In another embodiment, the lanthanide oxide is holmium oxide. Although this lanthanide oxide is as expensive as dysprosium oxide, it performs slightly better at least in ethane conversion to ethene in ODH reactions.

The present invention also relates to a catalyst, wherein component (B) is selected from the group consisting of alkali chlorides and/or earth alkali chlorides.

Preferred catalysts according to the present invention are catalysts comprising a eutectic mixture of components (A) and (B) preferably forming a shell, and preferably a closed shell, on the support (C). By "eutectic mixture" should be understood that the chlorides of the mixture should be present in the ratio at which the mixture solidifies at a lower temperature than any other composition (at a certain pressure). The presence of other salts, oxides... besides the chlorides forming a eutectic mixture together, should however not be excluded from the scope of the invention. The same applies to mixtures where the ratio is different from the eutectic ratio, especially if when heating them, a molten phase with the eutectic composition would form besides a solid phase.

In one embodiment, the present invention relates to a catalyst, wherein component (B) comprises (and preferably: consists in) RbCl and/or CsCl. The catalyst of this embodiment preferably comprises a eutectic mixture of LiCl (A) and RbCl (B) or of LiCl (A) and CsCl (B). Also preferably, these mixtures of (A) and (B) (namely: LiCl and RbCl or LiCl and CsCl) are comprised in or form a shell, and preferably a closed shell, on the support (C).

In another embodiment, component (B) comprises at least two chlorides (and preferably: at least 3) being different from LiCl and being preferably selected from the group consisting of alkali chlorides and/or earth alkali chlorides. In certain embodiments, catalysts of the present invention therefore e.g. comprise a eutectic mixture of LiCl(0.555) and KCl(0.187) and RbCl(0.014) and CsCl(0.243), or LiCl(0.575) and KCl(0.133) and CsCl(0.292), or LiCl(0.565) and RbCl(0.285) and CsCl(0.055), or LiCl(0.504) and KCl(0.183) and NaCl(0.080) and RbCl(0.233), or LiCl(0.582) and CsCl(0.398) and SrCl₂(0.02), or LiCl(0.566) and NaCl(0.024) and RbCl(0.41), or LiCl(0.581) and NaCl(0.017) and CsCl(0.403), or LiCl(0.580) and KCl(0.400) and BaCl₂ (0.060), or LiCl (0.430) and KCl (0.240) and NaCl (0.330), whereby the number in brackets indicates the mole fraction of the respective salt (e.g. LiCl) in each of the described eutectic mixtures (e.g. LiCl and RbCl). Also preferably, these mixtures of (A) and (B) form a shell, and preferably a closed shell, on the support (C).

Catalysts of the present invention comprising a eutectic mixture of components (A) and (B), wherein the eutectic mixture of components (A) and (B) is a eutectic mixture of LiCl and KCl and BaCl₂, or LiCl and CsCl, or LiCl and RbCl are e.g. preferred embodiments of the catalyst of the present invention, particularly if the support (C) comprises lanthanum oxide. Also preferably, these mixtures of (A) and (B) form a shell, and preferably a closed shell, on the support (C). In ODH of ethane, high ethane conversion levels are achieved and ethene selectivities above 90%, and to some extent even above 95% can be obtained using these catalysts.

In a preferred embodiment, the catalyst of the present invention is a catalyst wherein components (A) and (B) are comprised in or form a shell on the support (C) which builds a core. The catalyst thus exhibits a core-shell structure, wherein the shell constitutes a coating on the support. The core is thus covered partially or preferably entirely by the shell. When the core is entirely covered by the shell, the shell is also termed to be a "closed shell" in the present invention. In one embodiment, the catalyst of the present invention is a catalyst, wherein components (A) and (B) are comprised in a closed shell on the support which builds a core.

Preferably, the shell of the catalyst of this embodiment of the invention consists of chlorides. In one embodiment the shell of the catalyst of the present invention thus e.g. consists of a eutectic mixture of components (A) and (B), wherein component (B) is one or more chloride(s) being different from LiCl and being selected from the group consisting of alkali chlorides and/or earth alkali chlorides.

The catalyst of the present invention, which may exhibit a core-shell structure, has for example a particle size from 100 nm to 100 µm.

Melting of the salts (chlorides (A) and (B)) comprised in the shell may also by accompanied by spreading of the molten salts on the support, thus forming a closed shell when the catalyst is employed in a catalytic reaction. A low melting point leads to the full coverage of the oxide core, thus leading to high selectivity.

The catalyst of the present invention is preferably a catalyst comprising components (A) and (B) on a support (C) which comprises a metal oxide being different from a lanthanide oxide, and being preferably MgO, ZrO₂, SiO₂ and/or TiO₂, MgO being more preferred. It can also be advantageous that the support is doped with said lanthanide metal oxide (i.e. contains a small amount of lanthanide metal oxide in its lattice). The lanthanide metal oxide is then preferably present in an amount that is sufficient to substantially increase e.g. the ethene yield in a process for ODH of ethane when compared with the yield that can be achieved with a catalyst comprising no lanthanide metal oxide in the support. A preferred catalyst of the invention is thus for instance a catalyst, wherein the support comprises MgO. The atomic ratio of Mg and the lanthanide metal comprised in the catalyst can be between 500:1 and 5:1, preferably between 200:1 and 25:1 or more preferably between 100:1 and 30:1. Most preferably it is about 50:1.

The total chloride concentration in the catalyst of the present invention can e.g. be 1-50 wt-%, preferably 10-30 wt-% and most preferably about 20 wt-% each based on the weight of the support. In preferred embodiments of the present invention the total chloride concentration in the catalyst is thus 10-30 wt-% based on the weight of the support.

In one embodiment, the catalyst of the present invention does not comprise chlorides other than alkali chlorides and/or earth alkali chlorides.

The present invention also relates to the use of a catalyst as described above, in a catalytic reaction in conditions such that the mixture of components (A) and (B) is molten. A particularly preferred embodiment of that use is one wherein said catalyst exhibits a core-shell structure and wherein the molten mixture of (A) and (B) is a eutectic mixture which constitutes or forms part of a closed shell on the support which builds a core (the former embodiment being preferred).

The catalyst of the present invention can be prepared by putting the support in a bath of the mixture of (A) and (B) in molten state. Such a method is however limited to monolithic supports (as opposed to those in particulate form).

Alternatively, the catalyst of the present invention can be prepared by impregnating the support in an aqueous phase, or by simply milling the support and the chloride together.

The catalyst of the present invention can be prepared in analogy to the methods as known by the skilled person by preparing a dispersion of support materials in an aqueous phase and adding the metal nitrates as precursors (termed "nitrate method" for the purpose of the present invention) and HCl/NH₄Cl as chloride source, followed by evaporative separation from water, drying and calcination for 12 h at 650°C-700°C in synthetic air. However, this process has several disadvantages. The use of HCl can cause HCl losses while adding it to the synthesis mixture. This can cause wrong metal chloride ratios during the further production of the catalysts.

Thus, as a further aspect, the present invention also relates to a novel process for the manufacture of a catalyst of the present invention (as described above). The process is described in the claims. The invention thus relates to a process for the manufacture of a catalyst of the present invention, said process comprising
(a) mixing components (A) and (B) with an aqueous dispersion of the support (C) and of the lanthanide oxide,
(b) separating the water from the mixture, and
(c) calcining the residue obtained after separation from water.

For the purpose of the present invention, the novel process is also termed "chloride method" herein.

The catalyst prepared according to the above described method e.g. shows high ethene selectivities. The new process can also benefit from more exact metal/chloride ratios than obtained with the previously known process. Additional sources of chloride in the process for manufacture can be avoided, which is particularly beneficial for large scale industrial applications, e.g. in order to simplify the manufacturing process.

The mixing can for instance take place at room temperature (20-25°C) or at an elevated temperature from 50 to 150 °C, such as e.g. 80°C and the mixing time can be e.g. from 0.5 to 10 h, preferably between 1 to 5 h. Usually, it is sufficient to mix for 2 h, or even less. From an industrial point of view, this time can be adjusted to the minimum required.

In step (b) of the process of the second aspect of the present invention, the water is separated from the mixture obtained in step (a). This is usually done by evaporating the water from the mixture. For instance, evaporation of the water is conducted under reduced pressure and at elevated temperatures. In one embodiment of the present invention, the water is e.g. evaporated under reduced pressure at 50°C and the obtained residue is then dried, e.g. at an elevated temperature (e.g. 120°C) for several hours (e.g. 12 h).

The process of the present invention can also optionally include a calcination step to enable a better distribution of the chloride phase over the support. Preferably, the calcination step takes place at a temperature above the melting point of the (preferably eutectic) chloride mixture. A temperature above 500°C, or even above 600°C is preferred. Good results have been obtained at a temperature of about 650°C. In this case, the duration of calcination is typically in the range of hours, namely generally from 4 to 24 h, more preferably from 10 to 15 h. It can further be advantageous to conduct calcination in a stream of air. In some embodiments, the residue obtained from step (b) is crushed and/or sieved before calcining or after calcining or before and after calcining. Calcining is furthermore preferably conducted at 400-800°C, more preferably at 550-650°C. Most preferred is calcination at about 650°C, preferably over several hours.

In the process for manufacture of a catalyst of the present invention, it is preferred to use and aqueous dispersion of the support (C) and of the lanthanide oxide in step (a) to which each chloride is added directly or e.g. in form of aqueous solutions. Another alternative is to add the lanthanide oxide a in step (a) directly or as an aqueous dispersion to a container holding the one or more alkali chlorides and/or earth alkali chlorides in an aqueous medium.

In the process for manufacture of a catalyst of the present invention, preferably no precursors are used i.e. no HCl and no NH₄Cl are used, and preferably also, no nitrate(s) are used but instead: the metal/alkali chlorides are used right away for impregnating the support.

Catalysts obtained by the novel process of the present invention are preferred catalysts of the present invention.

According to another aspect, the present invention concerns a process for catalytic oxidative dehydrogenation (ODH) as described in the claims. The present invention thus relates to a process for catalytic ODH using a catalyst of the present invention. In a preferred embodiment, the present invention relates to a process for catalytic oxidative dehydrogenation of C2-C4 alkanes, particularly ethane and propane using a catalyst of the present invention.

A particularly preferred embodiment of the process of the third aspect of the present invention is a process for catalytic ODH of ethane to ethene using a catalyst of the present invention. The term catalytic oxidative dehydrogenation of ethane, is understood to mean a partial oxidation of ethane by oxygen in the presence of the above mentioned catalyst.

The process for catalytic ODH, particularly of ethane to ethene, may take place either at a temperature above 650°C and below the range of thermal cracking temperatures, or at a temperature less than or equal to 650°C. It is preferred that the process for catalytic ODH, particularly of ethane to ethene, using a catalyst of the present invention takes place at temperatures of less than or equal to 650°C. Lower reaction temperatures are desired, as unselective side reactions (radical-based total oxidation of hydrocarbons) can be suppressed.

The present invention also concerns a process for catalytic ODH wherein a chlorine containing hydrocarbon is present in the gas phase contacting the catalyst during the process. In one embodiment, the present invention thus also relates to a process for catalytic ODH, preferably of ethane to ethene, using a catalyst of the present invention, in which 1,2-dichloroethane (DCE) is present in the gas phase contacting the catalyst during the process, in particular in an amount up to 1000 ppm. It is namely so that some processes for manufacturing DCE from ethene integrate a step of making at least part of said ethene by ODH of ethane and include a step of recycling at least part of the unconverted ethane which is separated from the DCE, to the ODH stage.

Hence, according to a another aspect, the present invention concerns a process for the manufacture of 1,2-dichloroethane (DCE) comprising an ODH step of ethane to ethene as described above, a subsequent chlorination and/or oxychlorination step of the ethene and preferably, a recycling step of the unconverted ethane to the ODH step.

In a first sub-embodiment, the invention thus relates to a process for the manufacture of DCE starting from a stream of ethane according to which:
(a) the stream of ethane is subjected to an ODH step as described above producing a gas mixture containing ethene, unconverted ethane, water and secondary constituents;
(b) said gas mixture is optionally washed and dried thus producing a dried gas mixture;
(c) after an optional additional purification step, the dried mixture is then conveyed to a chlorination reactor supplied with a flow of chlorine so that at least 10% of the ethene is converted to 1,2-dichloroethane;
(d) the 1,2-dichloroethane formed in the chlorination reactor is optionally isolated from the stream of products derived from the chlorination reactor;
(e) the stream of products derived from the chlorination reactor, from which the 1,2-dichloroethane has optionally been extracted, is conveyed to an oxychlorination reactor in which the majority of the balance of ethene is converted to 1,2-dichloroethane, after optionally having subjected the latter to an absorption/desorption step e'), during which the 1,2-dichloroethane formed in the chlorination reactor is optionally extracted if it has not previously been extracted;
(f) the 1,2-dichloroethane formed in the oxychlorination reactor is isolated from the stream of products derived from the oxychlorination reactor and is optionally added to the 1,2-dichloroethane formed in the chlorination reactor;
(g) the stream of products derived from the oxychlorination reactor, from which the 1,2-dichloroethane has been extracted, optionally containing an additional stream of ethane previously introduced in one of steps b) to f), is optionally recycled to step a) after having been optionally purged of gases and/or after an optional additional treatment in order to eliminate the chlorinated products contained therein.

Such a process is described in the patent application WO 2008/000705 to the Applicant, the content of which is incorporated by reference in the present application.

In a second sub-embodiment, the invention relates to a process for the manufacture of DCE starting from a stream of ethane according to which:
(a) the stream of ethane is subjected to an ODH step as described above producing a gas mixture containing ethene, unconverted ethane, water and secondary constituents;
(b) said gas mixture is optionally washed and dried thus producing a dried gas mixture;
(c) after an optional additional purification step, said dried gas mixture is subjected to an absorption A1 which consists of separating said gas mixture into a fraction enriched with the compounds that are lighter than ethene containing some of the ethene (fraction A) and into a fraction F1;
(d) fraction A is conveyed to a chlorination reactor in which most of the ethene present in fraction A is converted to 1,2-dichloroethane and optionally the 1,2-dichloroethane obtained is separated from the stream of products derived from the chlorination reactor;
(e) optionally the stream of products derived from the chlorination reactor, from which the 1,2-dichloroethane has optionally been extracted, is subjected to an absorption A2 which consists of separating said stream into a fraction enriched with ethane F2 which is then conveyed back to fraction F1, and into a fraction enriched with compounds that are lighter than ethane F2';
(f) fraction F1, optionally containing fraction F2 recovered in step e) of absorption A2, is subjected to a desorption D which consists of separating fraction F1 into a fraction enriched with ethene (fraction B) and into a fraction F3, optionally containing the 1,2-dichloroethane formed in the chlorination reactor then extracted if it has not been extracted previously, which is recycled to at least one of the absorption steps, optionally after an additional treatment intended to reduce the concentration of compounds that are heavier than ethane in fraction F3;
(g) fraction B is conveyed to an oxychlorination reactor in which most of the ethene present in fraction B is converted into 1,2-dichloroethane, the 1,2-dichloroethane obtained is separated from the stream of products derived from the oxychlorination reactor and is optionally added to the 1,2-dichloroethane formed in the chlorination reactor; and the stream of products derived from the oxychlorination reactor, from which the 1,2-dichloroethane has been extracted, optionally containing an additional stream of ethane previously introduced in one of steps b) to g), is optionally recycled to step a) after having been optionally purged of gases and/or after an optional additional treatment in order to eliminate the chlorinated products contained therein.

Such a process is described in the patent application WO 2008/000702 to the Applicant, the content of which is incorporated by reference in the present application.

In a third sub-embodiment, the invention relates to a process for the manufacture of 1,2-dichloroethane starting from a stream of ethane according to which:
(a) the stream of ethane is subjected to an ODH step as described above producing a gas mixture containing ethene, unconverted ethane, water and secondary constituents;
(b) said gas mixture is optionally washed and dried thus producing a dried gas mixture;
(c) after an optional additional purification step, said dried gas mixture comprising the stream of products derived from the chlorination reactor R2 separated in step e) is subjected to an absorption A which consists of separating said gas mixture into a fraction enriched with the compounds that are lighter than ethene containing some of the ethene (fraction A) and into a fraction F 1;
(d) fraction A is conveyed to a chlorination reactor R1 in which most of the ethene present in fraction A is converted into 1,2-dichloroethane and the 1,2-dichloroethane obtained is separated from the stream of products derived from the chlorination reactor R1;
(e) fraction F1 is subj ected to a desorption D1 which consists of separating fraction F1 into an ethene fraction depleted of the compounds that are lighter than ethene (fraction C) which is conveyed to a chlorination reactor R2, the stream of products derived from this reactor being added to the dried gas mixture subjected to step c) after having optionally extracted the 1,2-dichloroethane formed, and into a fraction F2;
(f) fraction F2 is subjected to a desorption D2 which consists of separating fraction F2 into a fraction enriched with ethene (fraction B) and into a fraction F3, optionally containing the 1,2-dichloroethane formed in the chlorination reactor R2 then extracted, if it has not previously been extracted, which is recycled to the absorption A, optionally after an additional treatment intended to reduce the concentration, in fraction F3, of the compounds that are heavier than ethane;
(g) fraction B is conveyed to an oxychlorination reactor in which most of the ethene present in fraction B is converted into 1,2-dichloroethane, the 1,2-dichloroethane obtained is separated from the stream of products derived from the oxychlorination reactor and is optionally added to the 1,2-dichloroethane formed in the chlorination reactor R1 and optionally to that formed in the chlorination reactor R2; and the stream of products derived from the oxychlorination reactor, from which the 1,2-dichloroethane has been extracted, optionally containing an additional stream of ethane previously introduced in one of steps b) to g), is optionally recycled to step a) after having been optionally purged of gases and/or after an optional additional treatment in order to eliminate the chlorinated products contained therein.

Such a process is described in the patent application WO 2008/000693 to the Applicant, the content of which is incorporated by reference in the present application.

In the case a stream of ethane coming from a process according to any of the embodiments described above is recycled in step a) thereof (either in a continuous, loop process (which is generally the case in an industrial process) or in a batch one), the ODH step according to the invention is not detrimentally influenced by residual amounts of DCE in said stream.

The DCE obtained by (oxy)chlorination of ethene as described above may then be converted into VC. The invention therefore also relates to a process for the manufacture of VC. Namely, in this process, the 1,2-dichloroethane obtained as described above is subjected to a pyrolysis thus producing VC.

The conditions under which the pyrolysis may be carried out are known to a person skilled in the art. This pyrolysis is advantageously achieved by a reaction in the gas phase in a tube furnace. The usual pyrolysis temperatures extend between 400 and 600°C with a preference for the range between 480°C and 540°C. The residence time is advantageously between 1 and 60 seconds with a preference for the range of 5 to 25 seconds. The conversion rate of the DCE is advantageously limited to 45 to 75 % in order to limit the formation of by-products and fouling of the furnace pipes. The following steps make it possible, using any known device, to collect the purified VC and the hydrogen chloride to be upgraded preferably in the oxychlorination. Following purification, the unconverted DCE is advantageously reconveyed to the pyrolysis furnace.

In addition, the invention also relates to a process for the manufacture of PVC by polymerisation of the VC obtained as described above.

The process for the manufacture of PVC may be a bulk, solution or aqueous dispersion polymerization process, preferably it is an aqueous dispersion polymerization process.

The expression "aqueous dispersion polymerization" is understood to mean radical polymerization in aqueous suspension and also radical polymerization in aqueous emulsion and polymerization in aqueous microsuspension.

The expression "radical polymerization in aqueous suspension" is understood to mean any radical polymerization process performed in aqueous medium in the presence of dispersants and oil-soluble radical initiators.

The expression "radical polymerization in aqueous emulsion" is understood to mean any radical polymerization process performed in aqueous medium in the presence of emulsifiers and water-soluble radical initiators.

The expression "polymerization in aqueous microsuspension", also called polymerization in homogenized aqueous dispersion, is understood to mean any radical polymerization process in which oil-soluble initiators are used and an emulsion of monomer droplets is prepared by virtue of a powerful mechanical stirring and the presence of emulsifiers.

In relation to a similarly simplified thermal cracking process, the process according to the invention making use of an ODH step has the advantage of combining an endothermic step (ethane converted into ethene) with an exothermic water production step, of taking place at a moderate temperature and of avoiding having to provide the heat of reaction at a high temperature.

The process according to the invention also has the advantage of making it possible to recycle the stream of products derived from the oxychlorination, from which the DCE has been extracted, to the ODH step, thus ensuring an increased conversion of ethane into ethene. Furthermore, given the moderate temperature of the ODH relative to thermal cracking, even if this recycled stream contains traces of chlorinated organic products such as DCE, their presence does not cause material behaviour and corrosion problems as occur in the case of thermal cracking above 800°C. The presence of chlorinated products may furthermore be advantageous in so far as it allows an increase of the efficiency of the ODH reaction.

The process according to the invention has the advantage of not generating (starting from ethane) compounds comprising at least 3 carbon atoms in troublesome amounts, these compounds generally being responsible for a certain inhibition during the pyrolysis of the DCE. This inhibition is due to the formation of derivatives such as 1,2-dichloropropane and monochloropropenes. Their aptitude for forming stable allyl radicals explains their powerful inhibitory effect on the pyrolysis of DCE which is carried out by the radical route. The formation of these by-products containing 3 carbon atoms and heavier by-products furthermore constitutes an unnecessary consumption of reactants in the oxychlorination and in the chlorination, or generates costs for destroying them. Furthermore, these heavy compounds contribute to the soiling of the columns and evaporators.

The process of the invention also has the advantage of generating less acetic acid compared to prior art processes using catalysts active at a lower temperature.

Since the ODH reaction takes place at a lower temperature than thermal cracking, the process according to the invention is advantageously characterized, in addition, by the fact that the formation of heavy compounds by oligomerization is much lower.

The process according to the invention making use of an ODH step also has the advantage of allowing a limited conversion by passing to the ODH without having to resort to expensive separations such as those that require an ethene distillation.

Another advantage of the process according to the invention is that it makes it possible to have, on the same industrial site, a completely integrated process ranging from the hydrocarbon source - namely ethane - up to the polymer obtained starting from the monomer manufactured.

While different embodiments of the present invention have been described herein, the present invention is not limited to any specifically described embodiment. Rather any combination of the described features is intended to be encompassed by the present invention.

The present invention will now be described by the following examples which are not intended as being limiting.

### Catalyst preparation

### Chloride method

For the synthesis of catalysts according to the chloride method alkali/earth alkali chlorides were added to a suspension of MgO and La₂O₃ in 100 mL deionized water (masses of starting materials for the catalyst syntheses are given in Table 1). After stirring (stirrer speed: 500 min-1) at 80 °C for 2 h, the water was removed under reduced pressure (-70 mbar) at 50 °C. The obtained solids were dried for 12 h at 120 °C. Subsequently, the precursors were calcined for 12 h in synthetic air (100 mL/min) at 650°C.

**Table 1**

| | MgO [g] | Dy2O3 [g] | La2O3 [g] | LiCl [g] | RbCl [g] | BaCl2 [g] | CsCl [g] | NaCl [g] | KCl [g] | SrCl2*6 H2O [g] |
|---|---|---|---|---|---|---|---|---|---|---|
| LiCsCl/MgO-La2O3 | 6.64 | | 0.52 | 1.005 | | | 2.741 | | | |
| LiNaCsCl/MgO-La2O3 | 6.64 | | 0.52 | 0.983 | | | 2.714 | 0.04 | | |
| LiKCsCl/MgO-La2O3 | 6.64 | | 0.52 | 0.975 | | | 1.966 | | 0.397 | |
| LiNaRbCl/MgO-La2O3 | 6.64 | | 0.52 | 0.96 | 1.983 | | | 0.056 | | |
| LiRbCsCl/MgO-La2O3 | 6.64 | | 0.52 | 0.958 | 1.862 | | 0.337 | | | |
| LiNaKRbCl/MgO-La2O3 | 6.64 | | 0.52 | 0.855 | 1.127 | | | 0.187 | 0.546 | |
| LiKRbCsCl/MgO-La2O3 | 6.64 | | 0.52 | 0.941 | 0.068 | | 1.636 | | 0.558 | |
| LiNaKCl/MgO-La2O3 | 6.64 | | 0.52 | 0.729 | | | | 0.771 | 0.716 | |
| LiKBaCl/MgO-La2O3 | 6.64 | | 0.52 | 0.916 | | 0.5 | | | 1.193 | |
| LiSrCsCl/MgO-La2O3 | 6.64 | | 0.52 | 0.987 | | | 2.68 | | | 0.213 |

### Nitrate method

Catalysts according to the nitrate method were prepared by suspending MgO (6.64 g, 0.166 mol) and the rare earth metal oxide in 100 mL deionized water. Subsequently, alkali/earth alkali nitrate(s), were added to the mixture (masses of starting materials for the catalyst syntheses are given in Table 2). Then, the chloride was introduced by the addition of NH₄Cl (1.02 g, 0.019 mol) and HCl (37%, 1.57 mL). The atomic ratios of were chosen to obtain eutectic mixtures of the alkali/earth alkali chlorides. The suspensions obtained were stirred (stirring velocity: 500 min―1) at 80°C for 2 h, before the water was evaporated under reduced pressure (-70 mbar) at 50°C. Afterwards, the residues were dried for 12 h at 120°C. Then, the catalyst precursors were calcined for 12 h in synthetic air (100 mL/min) at 650°C.

**Table 2: Precursors in nitrate preparation method (different dopants)**

| Catalyst | Support [g] | | | | Precursor [g] | | Chloride source | |
|---|---|---|---|---|---|---|---|---|
| | MgO | Dy₂O₃ | La₂O₃ | Nd₂O₃ | LiNO₃ | RbNO₃ | HCl 37% [ml] | NH₄Cl{g] |
| Li-Rb-Cl/MgDyO | 6,64 | 0,6 | | | 1,51 | 2,65 | 1,6 | 1,04 |
| Li-Rb-Cl/MgLaO | 6,64 | | 0,52 | | 1,51 | 2,65 | 1,6 | 1,04 |
| Li-Rb-Cl/MgNdO | 6,64 | | | 0,54 | 1,51 | 2,65 | 1,6 | 1,04 |

### Catalytic ODH

Experiments were carried out in a continuous-flow fixed-bed reactor system. The educt gas stream was passed through a quartz tube (OD: 6mm; ID: 4 mm) surrounded by the reactor block, a heating coil and insulation material. The quartz tube was packed in the following order: Quartz wool, SiC, 300 mg catalyst (particle size: 300-600 µm) and 500 mg SiC, SiC, Quartz wool. Silicon carbide was added in order to improve the thermal conductivity and thus avoid hot spots and to minimize temperature differences in the catalyst bed. The reactor was heated with a heating coil. The temperature was measured using a thermocouple attached in-between the two halves of the reactor. Mass flow controllers were utilized to control the flow rates of the gases having passed a filter. By means of valves the gas stream could be fed to the reactor or to the vent. The product gas stream was analyzed by a Maxum edition II Process gas chromatograph (Siemens) equipped with TCD detectors. For the separation of oxygen, carbon monoxide and methane a Molesieve 5A column was employed. The other hydrocarbons were separated by a HayeSep Q column and a HayeSep T precolumn. A Poraplot Q column was applied for the additional detection of products.

Reactant molar ratio / %: He/O₂/C₂H₆ = 86.0/7.0/7.0 (total flow: 11.52 mL/min), Pressure: 1 bar

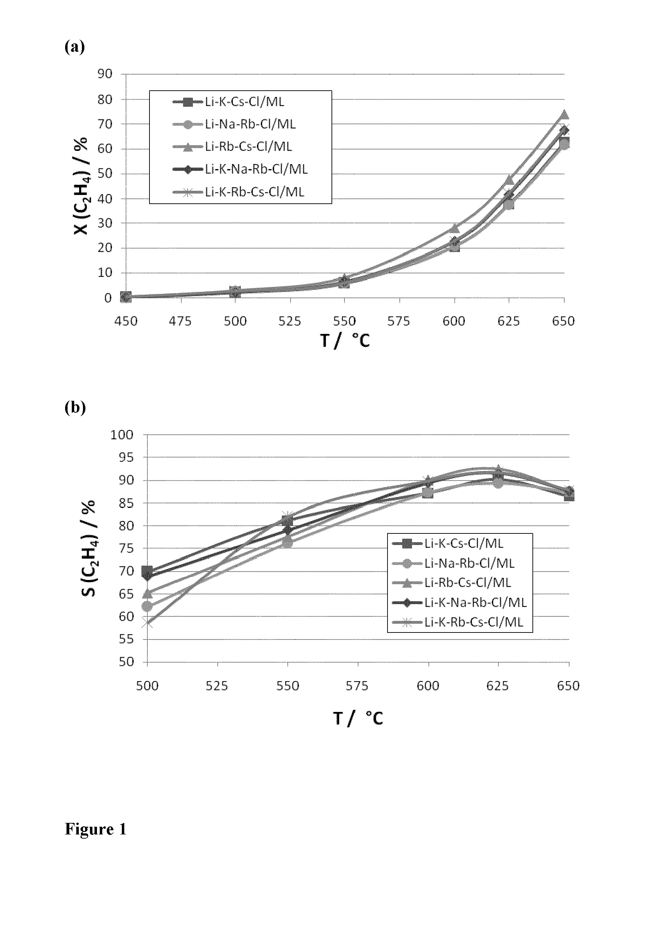

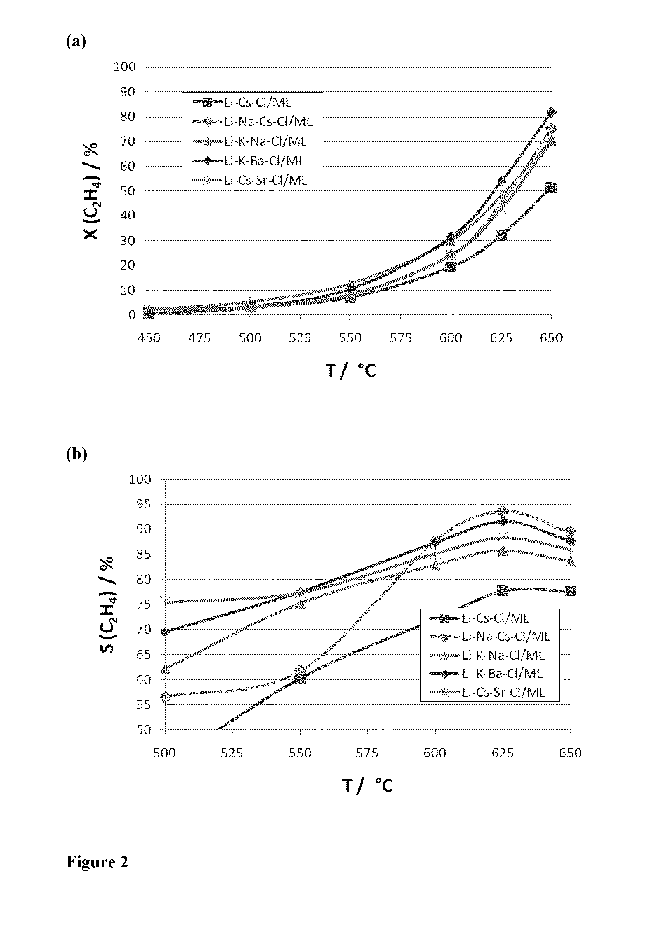

Ethane conversion, ethene yield and ethene selectivity were tested for different catalysts prepared by the nitrate method or chloride method. Ethane conversion and ethene selectivity were also assessed in the presence or absence of 1000 ppm 1,2-dichloroethane. Test results of the different catalysts in ODH of ethane are shown in Figures 1-3 and 5, and Figure 4 respectively.

### Description of the drawings

**Figure 1** shows performance (ethene conversion (a), ethene selectivity (b)) of different La₂O₃-MgO (ML) supported catalysts in ODH of ethane. Catalysts were prepared according to the chloride method (see Table 1).
**Figure 2** shows performance (ethene conversion (a), ethene selectivity (b)) of different La₂O₃-MgO (ML) supported catalysts in ODH of ethane. Catalysts were prepared according to the chloride method (see Table 1).
**Figure 3** shows performance (ethane conversion (a), ethene selectivity (b)) of Dy₂O₃-MgO, La₂O₃-MgO or Nd₂O₃-MgO supported catalysts containing LiCl and RbCl in ODH of ethane. Catalysts were prepared according to the nitrate method (see Table 2).
**Figure 4** shows performance (ethane conversion (a), ethene selectivity (b)) of a La₂O₃-MgO (ML) supported catalyst containing LiCl and RbCl in ODH of ethane in the presence or absence of 1,2-dichloroethane. Catalysts were prepared according to the nitrate method (see Table 2).
**Figure 5** is the equivalent to figure 3 completed with performance data of catalysts including other rare earth dopants than Dy, La or Nd, namely with Ho, Sm, Ce, Tb and Pr.

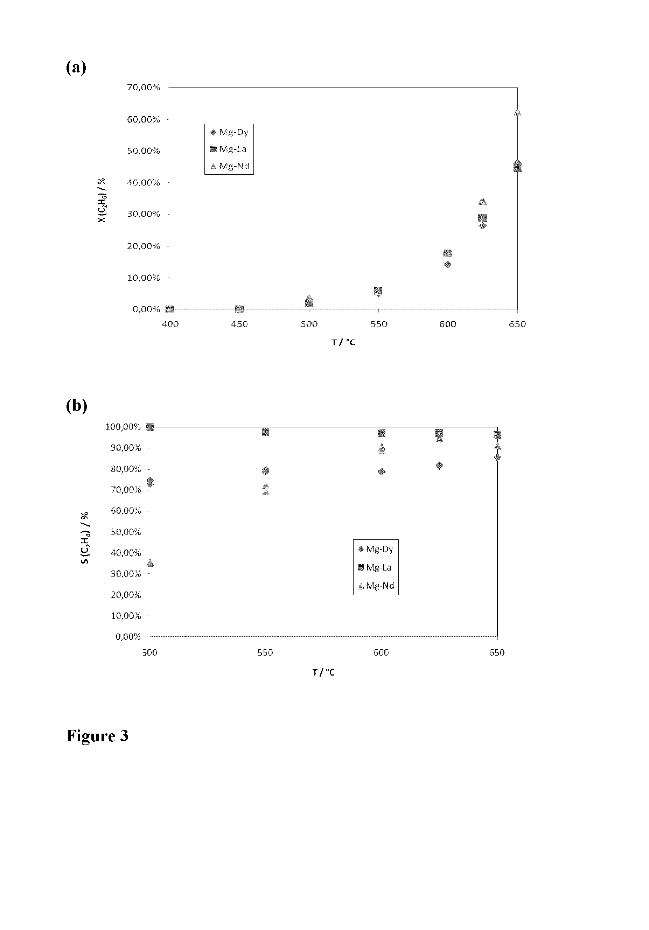

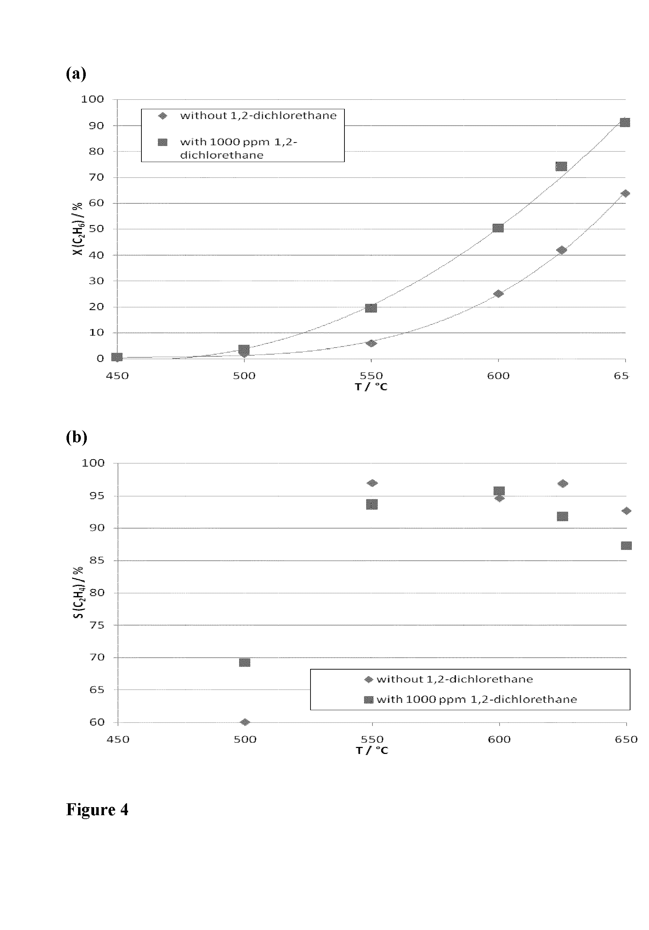

Figures 1 to 5 show the high efficiency of the new catalyst. High efficiency of the catalysts is expressed in high conversion rates or ethane, high ethene yields and high ethene selectivities. While e.g. reaching high conversion levels, ethene selectivities around or even above 90% can be obtained. For examples, the production of ethene with a selectivity of around 95% at an ethene yield around 50% at 625°C is enabled with a catalyst of the present invention. Figure 3 also demonstrates that a catalyst according to the invention, which comprises a lanthanum containing compound or a neodymium containing compound provides for e.g. higher ethene selectivities at comparable or higher ethane conversion levels when compared to a catalyst (not according to the invention) comprising a more expensive dysprosium containing compound. The catalyst comprising the lanthanum containing compound showed particularly high ethene selectivities. Moreover, the examples demonstrate that when using a catalyst of the present invention in ODH of ethane the presence of 1,2-dichloroethane can boost ethane conversion in a temperature range between 450°C and 650°C, with ethane conversion raised to above 90% at 650°C compared to approximately 60% ethane conversion in the absence of 1,2-dichloroethane. Ethene selectivities are not noticeable influenced up to a temperature of 600°C (Figure 4).

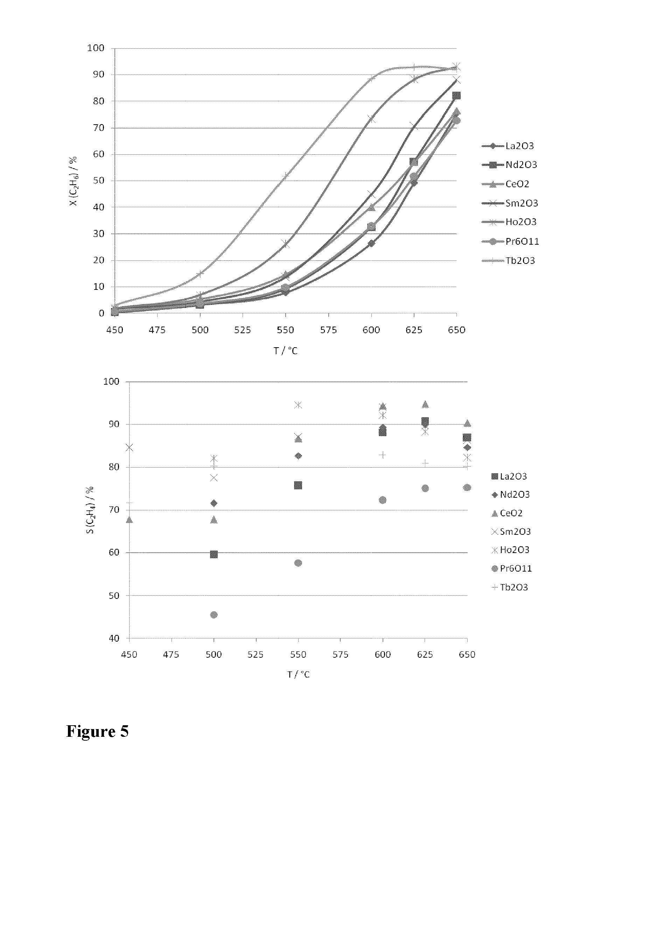

Figure 5 shows that all lanthanides are performing well in terms of ethane conversion and that while Tb and Pr are less effective in terms of selectivity, Ho is performing very well both in terms of ethane conversion and selectivity. It also shows that cheaper materials like La, Nd, Sm and Ce are performing quite well, while being much less expensive than Dy and Ho.

## Claims

1. A catalyst comprising LiCl (A) and one or more chlorides being different from LiCl (B) on a support (C) which comprises a lanthanide oxide different from dysprosium oxide.

2. Catalyst according to claim 1, wherein the lanthanide oxide is selected from lanthanum oxide, neodymium oxide, samarium oxide, cerium oxide and/or holmium oxide.

3. Catalyst according to claim 2, wherein the lanthanide oxide is selected from lanthanum oxide, neodymium oxide, samarium oxide and/or cerium oxide, lanthanum oxide being preferred.

4. Catalyst according to claim 3, wherein the lanthanide oxide is selected from lanthanum oxide and/or a neodymium oxide.

5. Catalyst according to claim 2, wherein the lanthanide oxide is holmium oxide.

6. The catalyst of any of the preceding claims, wherein component (B) is selected from the group consisting of alkali chlorides and/or earth alkali chlorides.

7. The catalyst of any of the preceding claims comprising a eutectic mixture of components (A) and (B).

8. The catalyst of any of the preceding claims, wherein components (A) and (B) are comprised in or form a shell on the support which builds a core.

9. The catalyst of the preceding claim, wherein the shell is a closed shell.

10. The catalyst of any of the preceding claims wherein the support comprises a metal oxide being different from a lanthanide, and being preferably MgO.

11. The catalyst of any of the preceding claims, wherein the total chloride concentration is 1-50 wt-%, preferably 10-30 wt-% and most preferably about 20 wt-% each based on the weight of the support.

12. Use of a catalyst of any of the preceding claims, in a catalytic reaction in conditions such that the mixture of components (A) and (B) is molten.

13. The use of the preceding claim, wherein the catalyst exhibits a core-shell structure and wherein the molten mixture is a eutectic mixture which is comprised in or forms part of a closed shell on the support which builds a core.

14. Process for catalytic oxidative dehydrogenation (ODH) using a catalyst of any of claims 1-11.

15. Process of claim the preceding claim for catalytic ODH of ethane to ethene.
